# FASCICULE DE BREVET EUROPEEN

(11) **EP 0 901 520 B1**
(45) Date de publication et mention de la délivrance du brevet: **07.04.2004**
(21) Numéro de dépôt: 97918215.1
(22) Date de dépôt: 14.04.1997
(51) Int. Cl.: C12N 1/20

(54) **MILIEU DE CULTURE POUR LA MISE EN EVIDENCE DES BACTERIES E. COLI ENTEROHEMORRAGIQUES, ET PROCEDE POUR SA MISE EN EVIDENCE**
WACHSTUMSMEDIUM ZUM NACHWEIS VON ENTEROHAEMORRAGISCHE E. COLI-BAKTERIEN, UND NACHWEISVERFAHREN
CULTURE MEDIUM FOR REVEALING ENTEROHAEMORRAGIC E. COLI BACTERIA AND PROCEDURE THEREFOR

(30) Priorité: 15.04.1996 FR 9604655
(43) Date de publication de la demande: 17.03.1999
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: PCT/FR1997/000656
(87) Numéro de publication internationale: WO 1997/039103

(56) Documents cités:
- WO-A-92/02820
- WO-A-94/08043
- WO-A-95/04157
- FR-A- 2 696 476
- US-A- 5 210 022
- JOURNAL OF THE AMERICAN SOCIETY OF BREWING CHEMISTS, vol. 51, no. 4, 1993, USA, pages 185-187, XP000196712 T. D'AMORE ET AL.: "X-alpha-Gal medium"
- DATABASE WPI Week 9214 Derwent Publications Ltd., London, GB; AN 92-110005 XP002020743 & JP 04 051 900 A ((NISR) NISSUI PHARM KK) , 20 Février 1992
- JOURNAL OF FOOD PROTECTION, vol. 51, no. 5, 1988, USA, pages 402-404, XP000196707 E.W. FRAMPTON: "Evaluation of the beta-glucuronidase substrate 5-bromo-4-chloro-3-indolyl-beta-D-glucuron ide (X-GLUC) in 24-hour direct plating method for Escherichia coli"
- LETTERS IN APPLIED MICROBIOLOGY, vol. 13, 1991, GB, pages 212-215, XP000196715 I.D. OGDEN AT AL.: "An evaluation of fluorogenic and chromogenic assays for the direct enumeration of Escherichia coli"
- APPLIED AND ENVIRONMENTAL MICROBIOLOGY, vol. 53, no. 10, 1987, USA, pages 2394-2396, XP000196714 M.P. DOYLE ET AL.: "Isolation of Escherichia coli O157:H7 from retail fresh meats and poultry"
- APPLIED AND ENVIRONMENTAL MICROBIOLOGY, vol. 54, no. 10, 1988, USA, pages 2536-2540, XP000196713 E.C.D. TODD ET AL.: "Rapid hydrophobic grid membrane filter-enzyme-labeled antibody procedure for identification and enumeration of Escherichia coli O157 in foods"

## Description

La présente invention concerne un nouveau milieu d'isolement et d'identification des bactéries *E. Coli* O157, et un procédé pour leur mise en évidence.

*Escherichia coli* entérohémorragique (EHEC), en particulier le sérotype 0157, a été à l'origine de la plupart des cas d'intoxications alimentaires aux Etats-Unis, au Canada et en Europe. En Amérique du Nord, la viande de boeuf (crue notamment), les produits à base de boeuf, le lait cru, ont été impliqués dans ces épidémies.

Les caractéristiques de virulence de ce germe, identiques à celles des *E coli* entéropathogènes et des *E. coli* vérotoxinogènes font de ce germe un problème majeur de santé publique. En effet, il provoque des colites hémorragiques caractérisées par des diarrhées sanguinolentes, symptômes pouvant évoluer vers des complications rénales très graves (syndrôme urémique hémolytique).

Les recherches s'orientent donc, ces dernières années, vers la détection rapide des souches EHEC dans les produits alimentaires (FDA, 8th édition, 1995 - Bolton et al., 1995), et plus particulièrement vers une détection spécifique du sérotype O157.

Les milieux d'isolement de l'art antérieur sont :
- en particulier des milieux peu sélectifs, tels que le milieu MacConkey au sorbitol et examen de toutes les bactéries gram négatives, sorbitol négatives,
- on a parfois ajouté des antibiotiques avec un succès limité car on peut obtenir une inhibition de la croissance des *E. coli* O157 recherchés,
- on a parfois ajouté un composé fluorogène ou chromogène permettant d'éliminer les colonies de bactéries possédant une β-glucuronidase (essentiellement des *E. coli* typiques).

La plupart des souches d'*Escherichia coli* O157 isolées d'épidémies ne fermente pas le sorbitol en 24 heures, n'a pas d'activité β-glucuronidase et ne se développe pas à 45,5°C. Aussi, beaucoup de méthodes de screening des souches EHEC d'échantillons cliniques ou alimentaires utilisent le sorbitol MacConkey agar (SMAC) comme milieu d'isolement primaire.

Les colonies suspectes sorbitol-négatif sont ensuite confirmées comme *Escherichia coli* O157 par tests biochimiques et sérologiques.

Parmi les autres milieux les plus couramment utilisés citons :
→ CT-SMAC : milieu dérivé du SMAC, contenant deux inhibiteurs (céfixime et tellurite de potassium) le rendant plus sélectif. En effet, ces composés inhibent de nombreuses bactéries ne fermentant pas le sorbitol, telles que *Proteus* spp, *Morganella morganii, Providencia* spp, *Hafnia alvei* .... Une étude réalisée en 1993 a montré que près de 400 souches d'*E*. *coli* O157 EHEC, toutes cultivaient sur le milieu CT-SMAC, permettant la mise en évidence des colonies sorbitol-négatif.
→ CR-SMAC : milieu dérivé du SMAC, contenant du céfixime et du rhamnose, pour la mise en évidence des colonies sorbitol-négatif, rhamnose-négatif.
→ SMAC + MUG (4-méthylhumbelliféryl-β-D-glucuronide), pour la mise en évidence des colonies sorbitol-négatif, β-glucuronidase-négatif.
→ SMAC + BCIG (5-bromo-4-chloro-3-indoxyl-D-glucuronide), pour la mise en évidence des colonies sorbitol-négatif, β-glucuronidase-négatif.
→ Gélose Fluorocult *E. coli* O157 : H7 : milieu contenant du sorbitol, du MUG et du thiosulfate de sodium, pour la mise en évidence des colonies sorbitol-négatif, MUG-négatif et H₂S négatif.

Les variantes du milieu SMAC, milieu peu sélectif au départ, ont été réalisées par addition d'inhibiteurs, contribuant à augmenter la sensibilité d'isolement d'*E. coli* O157 par élimination des flores associées.

Toutefois, les techniques de détection actuelles présentent des inconvénients majeurs, tant du point de vue de la sensibilité que de la spécificité, *E. coli* O157 étant présent en faible quantité dans les échantillons alimentaires, par rapport aux autres germes présents, et aux autres espèces telles que *E. hermanii* ayant les mêmes phénotypes sur les milieux actuels.

La présente invention apporte une solution à ce problème puisqu'il permet une sélectivité et une spécificité améliorée par rapport aux milieux de l'état de la technique, puisque l'addition dans un milieu incolore de chromogène détectant l'enzyme α-galactosidase permet de repérer rapidement et aisément les souches de sérogroupe O157. Nous obtenons des colorations bien concentrées, dont la couleur dépend de la partie chromophore du chromogène choisi, localisées au niveau des colonies et qui permettent un repérage aisé.

La présente invention concerne donc un nouveau milieu de culture pour la mise en évidence des bactéries *E*. *coli* entérohémorragiques, en particulier de sérotype O157, lequel consiste, outre un milieu de culture pour *E. coli*, en un composé chromogène substrat de l'enzyme α-galactosidase.

Pour augmenter la sélectivité, nous pouvons ajouter un composé chromogène substrat de la β-glucosidase qui permet d'éliminer en particulier un grand nombre de bactéries coliformes.

Pour augmenter la sélectivité, nous pouvons ajouter un composé chromogène substrat de la β-glucuronidase qui permet d'éliminer en particulier la plupart des *E. coli* des sérogroupes autres que O157 et O11.

D'une manière avantageuse, le composé chromogène substrat de l'enzyme α-galactosidase est choisi parmi les dérivés de l'indoxyle-α-galactoside, dont le chromophore est choisi parmi les radicaux indoxyle substitués ou non.

De préférence, le composé chromogène substrat de la β-glucosidase est choisi parmi les dérivés de l'indoxyle-β-glucoside, dont le chromophore est choisi parmi les radicaux indoxyle substitués ou non.

Le composé chromogène substrat de la β-glucuronidase est de préférence choisi parmi les dérivés de l'indoxyle-β-glucuronide, dont le chromophore est choisi parmi les radicaux indoxyle substitués ou non.

Par indoxyle substitué ou non, on entend les radicaux indoxyle et indoxyle substitué par un ou plusieurs radicaux alkyles ou halogènes. Il s'agit plus particulièrement des radicaux indoxyle alkylés, halogénés, di-halogénés ou tri-halogénés, de préférence choisis parmi les radicaux bromo-indoxyle, chloro-indoxyle, dichloro-indoxyle, chloro-bromo-indoxyle, tri-chloro-indoxyle et méthyl-indoxyle.

D'une manière avantageuse, les composés chromophores indoxylés sont choisis parmi les radicaux 3-indoxyle, 6-chloro-indoxyle, 5-bromo-indoxyle, 3-bromo-indoxyle, 4,6-dichloro-indoxyle, 6,7-dichloro-indoxyle, 5-bromo-4-chloro-indoxyle, 5-bromo-6-chloro-indoxyle ou 4,6,7-trichloro-indoxyle.

D'une manière préférentielle, le composé chromogène substrat de l'enzyme α-galactosidase est choisi parmi les composés suivants :
5-bromo-6-chloro-3-indoxyl-α-galactoside, et
6-chloro-3-indoxyl-α-galactoside.

Pour les composés chromogènes substrats des enzymes β-glucosidase et/ou β-glucuronidase, le chromophore est de préférence choisi parmi les radicaux suivants :
5-bromo-4-chloro-3-indoxyle,
5-bromo-3-indoxyle, et
3-indoxyle.

D'une manière avantageuse, le milieu selon l'invention comprend entre 0,01 et 0,2 g/l de chromogène substrat de l'α-galactosidase, de préférence environ 0,05 g/l.

Le cas échéant, la concentration des composés chromogènes substrats de la β-glucosidase et/ou β-glucuronidase est également comprise entre 0,01 et 0,2 g/l, de préférence environ 0,05 g/l.

D'autres caractéristiques des milieux selon l'invention apparaîtront à la lumière des exemples ci-dessous.

### Exemple 1 :

| | |
|---|---|
| Formulation CHROMagar O157 | en g/l |
| agar | 15 |
| peptone | 5 |
| extrait de levure | 2 |
| extrait de viande | 1 |
| NaCl | 5 |
| 5-bromo-6-chloro-3-indoxyl-α-galactoside | 0,05 |
| 5-bromo-4-chloro-3-indoxyl-β-glucoside | 0,05 |
| 5-bromo-4-chloro-3-indoxyl-β-glucuronide | 0,05 |
| désoxychotate | 0,5 |

### Exemple 2 : Colorations obtenues par différentes souches

On inocule le milieu de culture avec différentes souches de bactéries, puis on laisse incuber 24 heures.

| | Mac Conkey Sorbitol | CHROMagar O157 |
|---|---|---|
| Coliformes | rouge | bleu |
| *E coli* typique | rouge | bleu |
| *E coli* O157 | incolore | violet |
| *E. hermanii* | incolore | incolore |
| *Hafnia alvei* | incolore | incolore |
| *Proteus mirabilis* | incolore | incolore |
| *Pseudomonas* | incolore | incolore |

On trouve même des souches de *E*. *coli* O157 qui sont pourtant SLT+ (Shigella Like Toxine positives) avec le caractère sorbitol positif et qui sont détectées par le milieu de l'invention, tandis qu'elles sont faussement négatives, rouges sur milieu Mac Conkey sorbitol, et donc manquées. Le milieu de l'invention apporte donc non seulement un avantage de spécificité (élimination de faux positifs) mais apporte un avantage de sensibilité (détection de souches faussement négatives sur milieu Mac Conkey sorbitol) ce qui est très important.

De plus, le milieu de l'invention donne un bon rendement de croissance car, sauf de manière élective pour inhiber les bactéries gram positives, le milieu n'est pas fondé principalement sur un principe de croissance sélective.

La présente invention concerne également un procécé de mise en évidence de *E. coli* entérohémorragiques O157 dans un prélèvement, dans lequel on inocule le milieu de culture selon l'invention avec le prélèvement ou un inoculum dudit prélèvement, puis on détecte le cas échéant la présence de *E. coli* entérohémorragiques.

Dans le domaine de la microbiologie alimentaire, on recherche par exemple à démontrer qu'il y a moins de dix bactéries *E. coli* O157:H7 dans un aliment. On emploie une méthode de croissance microbienne puis éventuellement une méthode d'enrichissement par immunocapture IMS avant étalement, à l'aide d'anticorps accrochés sur des billes. Cependant, cet enrichissement n'élimine pas plusieurs bactéries qui donnent des faux positifs sur les milieux de l'art antérieur. L'utilisation du milieu selon l'invention apporte donc un avantage crucial même si l'on utilise cette méthode IMS d'enrichissement par immunocapture.

## Revendications

1. Milieu de culture pour la mise en évidence des bactéries *E. coli* entérohémorragiques de sérotype O157, **caractérisé en ce qu'**il consiste, outre en un milieu de culture pour *E. coli,* en un seul composé chromogène substrat de l'enzyme α-galactosidase.

2. Milieu de culture selon la revendication 1, **caractérisé en ce que** le composé chromogène substrat de l'enzyme α-galactosidase est choisi parmi les dérivés de l'indoxyl-α-galactoside, dont le chromophore est choisi parmi les radicaux indoxyle substitués ou non.

3. Milieu de culture selon la revendication 2, **caractérisé en ce que** le chromophore du composé chromogène substrat de l'enzyme α-galactosidase est choisi parmi les radicaux suivants :
5-bromo-6-chloro-3-indoxyl-α-galactoside, et
6-chloro-3-indoxyl-α-galactoside.

4. Milieu de culture selon l'une des revendications 1 à 3, **caractérisé en ce que** la concentration du composé chromogène substrat de l'α-galactosidase est comprise entre 0,01 et 0,2 g/l, de préférence environ 0,05 g/l.

5. Procédé de mise en évidence de *E. coli* entérohémorragiques O157 dans un prélèvement, **caractérisé en ce que** l'on inocule le milieu de culture selon l'une des revendications 1 à 4, avec le prélèvement ou en inoculum provenant dudit prélèvement et **en ce que** l'on détecte le cas échéant la présence de *E. coli* entérohémorragiques.

6. Procédé selon la revendication 5, **caractérisé en ce que** le milieu de culture comprend un composé chromogène substrat de la β-glucosidase et/ou un composé chromogène substrat de la β-glucuronidase.

7. Procédé selon la revendication 6, **caractérisé en ce que** le composé chromogène substrat de la β-glucosidase est choisi parmi les dérivés de l'indoxyle-β-glucoside et/ou le composé chromogène substrat de la β-glucuronidase est choisi parmi les dérivés de l'indoxyle-β-glucuronide, dont le chromophore est choisi parmi les radicaux indoxyle substitués ou non.

8. Procédé selon la revendication 7, **caractérisé en ce que** le chromophore du composé chromogène substrat de l'enzyme β-glucosidase et/ou du composé chromogène substrat de l'enzyme β-glucuronidase est choisi parmi les radicaux suivants :
5-bromo-4-chloro-3-indoxyle
5-bromo-3-indoxyle, et
3-indoxyle.

9. Procédé selon la revendication 8, **caractérisé en ce que** la concentration des composés chromogènes substrats de la β-glucosidase et/ou de la β-glucuronidase est comprise entre 0,01 et 0,2 g/l, de préférence 0,05 g/l.

10. Procédé selon l'une des revendications 5 à 9, **caractérisé en ce que** l'on emploie au préalable une méthode de croissance microbienne du prélèvement, puis éventuellement une méthode d'enrichissement par immunocapture IMS, avant d'inoculer le milieu de culture.

## Patentansprüche

1. Kulturmedium für den Nachweis von enterohämorrhagischen *E. coli*-Bakterien vom Serotyp O157, **dadurch gekennzeichnet, dass** es außer aus einem Kulturmedium für *E. coli* aus einer einzigen chromogenen Verbindung, welche ein Substrat des Enzyms α-Galactosidase ist, besteht.

2. Kulturmedium nach Anspruch 1, **dadurch gekennzeichnet, dass** die chromogene Verbindung, welche ein Substrat des Enzyms α-Galactosidase ist, ausgewählt wird unter den Derivaten von Indoxyl-α-galactosid, bei denen das Chromophor unter den substituierten oder nicht-substituierten Indoxylresten ausgewählt ist.

3. Kulturmedium nach Anspruch 2, **dadurch gekennzeichnet, dass** das Chromophor der chromogenen Verbindung, welche ein Substrat des Enzyms α-Galactosidase ist, ausgewählt wird unter den folgenden Resten:
5-Brom-6-chlor-3-indoxyl-α-galactosid und
6-Chlor-3-indoxyl-α-galactosid.

4. Kulturmedium nach einem der Ansprüche 1 bis 3, **dadurch gekennzeichnet, dass** die Konzentration der chromogenen Verbindung, welche ein Substrat von α-Galactosidase ist, zwischen 0,01 und 0,2 g/l einschließlich, vorzugsweise ungefähr 0,05 g/l beträgt.

5. Verfahren zum Nachweis von enterohämorrhagischen *E. coli* 0157 in einer Probe, **dadurch gekennzeichnet, dass** man das Kulturmedium nach einem der Ansprüche 1 bis 4 mit der Probe oder einem Inokulum, das von der Probe stammt, animpft und dass man gegebenenfalls das Vorhandensein von enterohämorrhagischen *E. coli* nachweist.

6. Verfahren nach Anspruch 5, **dadurch gekennzeichnet, dass** das Kulturmedium eine chromogene Verbindung, welche ein Substrat der β-Glucosidase ist, und/oder eine chromogene Verbindung, welche ein Substrat der β-Glucuronidase ist, umfasst.

7. Verfahren nach Anspruch 6, **dadurch gekennzeichnet, dass** die chromogene Verbindung, welche ein Substrat der β-Glucosidase ist, ausgewählt wird unter den Derivaten von Indoxyl-β-glucosid und/oder die chromogene Verbindung, welche ein Substrat der β-Glucuronidase ist, ausgewählt wird unter den Derivaten von Indoxyl-β-glucuronid, bei denen das Chromophor unter den substituierten oder nicht-substituierten Indoxylresten ausgewählt ist.

8. Verfahren nach Anspruch 7, **dadurch gekennzeichnet, dass** das Chromophor der chromogenen Verbindung, welche ein Substrat des Enzyms β-Glucosidase ist, und/oder der chromogenen Verbindung, welche ein Substrat des Enzyms β-Glucuronidase ist, ausgewählt wird unter den folgenden Resten:
5-Brom-4-chlor-3-indoxyl
5-Brom-3-indoxyl und
3-Indoxyl.

9. Verfahren nach Anspruch 8, **dadurch gekennzeichnet, dass** die Konzentration der chromogenen Verbindungen, welche Substrate der β-Glucosidase und/oder der β-Glucuronidase sind, zwischen 0,01 und 0,2 g/l einschließlich, vorzugsweise 0,05 g/l beträgt.

10. Verfahren nach einem der Ansprüche 5 bis 9, **dadurch gekennzeichnet, dass** man vorab ein Verfahren zur mikrobiellen Vermehrung der Probe, dann gegebenenfalls ein Verfahren zur Anreicherung durch IMS-Immuneinfang ("IMS-Immunocapture") einsetzt, bevor das Kulturmedium angeimpft wird.

## Claims

1. Culture medium for revealing enterohaemorrhagic *E. coli* bacteria of serotype 0157, **characterized in that** it consists of, besides a culture medium for *E. coli*, a single chromogenic compound which is a substrate for the α-galactosidase enzyme.

2. Culture medium according to Claim 1, **characterized in that** the chromogenic compound which is a substrate for the α-galactosidase enzyme is chosen from indoxyl-α-galactoside derivatives, in which the chromophore is chosen from substituted or unsubstituted indoxyl radicals.

3. Culture medium according to Claim 2, **characterized in that** the chromophore of the chromogenic compound which is a substrate for the α-galactosidase enzyme is chosen from the following radicals:
5-bromo-6-chloro-3-indoxyl-α-galactoside, and
6-chloro-3-indoxyl-α-galactoside.

4. Culture medium according to one of Claims 1 to 3, **characterized in that** the concentration of the chromogenic compound which is a substrate for α-galactosidase is between 0.01 and 0.2 g/l, preferably about 0.05 g/l.

5. Process for revealing enterohaemorrhagic *E. coli* 0157 in a sample, **characterized in that** the culture medium according to one of Claims 1 to 4 is inoculated with the sample or with inoculum derived from the said sample, and **in that** the presence, if any, of enterohaemorrhagic *E. coli* is revealed.

6. Process according to Claim 5, **characterized in that** the culture medium comprises a chromogenic compound which is a substrate for β-glucosidase, and/or a chromogenic compound which is a substrate for β-glucuronidase.

7. Process according to Claim 6, **characterized in that** the chromogenic compound which is a substrate for β-glucosidase is chosen from indoxyl-β-glucoside derivatives, and/or the chromogenic compound which is a substrate for β-glucuronidase is chosen from indoxyl-β-glucuronide derivatives, in which the chromophore is chosen from substituted or unsubstituted indoxyl radicals.

8. Process according to Claim 7, **characterized in that** the chromophore of the chromogenic compound which is a substrate for the β-glucosidase enzyme and/or of the chromogenic compound which is a substrate for the β-glucuronidase enzyme is chosen from the following radicals:
5-bromo-4-chloro-3-indoxyl,
5-bromo-3-indoxyl, and
3-indoxyl.

9. Process according to Claim 8, **characterized in that** the concentration of chromogenic compounds which are substrates for β-glucosidase and/or for β-glucuronidase is between 0.01 and 0.2 g/l, preferably 0.05 g/l.

10. Process according to one of Claims 5 to 9, **characterized in that** a method for microbial growth of the sample is used beforehand, optionally followed by a method of enrichment by IMS immunocapture, before inoculating the culture medium.
